(19) Europäisches Patentamt
European Patent Office
Office européen des brevets

(11) **EP 1 706 509 B1**

## (12) FASCICULE DE BREVET EUROPEEN

(45) Date de publication et mention
de la délivrance du brevet:
**05.11.2008 Bulletin 2008/45**

(21) Numéro de dépôt: **04816604.5**

(22) Date de dépôt: **23.12.2004**

(51) Int Cl.:
***C12Q 1/68*** (2006.01)

(86) Numéro de dépôt international:
**PCT/FR2004/050757**

(87) Numéro de publication internationale:
**WO 2005/068654 (28.07.2005 Gazette 2005/30)**

(54) **PUCE D'ANALYSE AVEC GAMME ETALON, TROUSSES ET PROCEDES D'ANALYSE**

ANALYSE-CHIP MIT REFERENZSKALA-KITS UND ANALYSEMETHODEN

ANALYSIS CHIP WITH REFERENCE SCALE KITS AND ANALYTICAL METHODS

(84) Etats contractants désignés:
**AT BE BG CH CY CZ DE DK EE ES FI FR GB GR HU IE IS IT LI LT LU MC NL PL PT RO SE SI SK TR**

(30) Priorité: **29.12.2003 FR 0351222**

(43) Date de publication de la demande:
**04.10.2006 Bulletin 2006/40**

(73) Titulaires:
• **COMMISSARIAT A L'ENERGIE ATOMIQUE**
**75015 Paris (FR)**
• **BIOMERIEUX SA**
**69280 Marcy l'Etoile (FR)**

(72) Inventeurs:
• **GINOT, Frédéric**
**F-38120 SAINT EGREVE (FR)**
• **NOVELLI-ROUSSEAU, Armelle**
**F-38180 SEYSSINS (FR)**
• **MALLARD, Frédéric**
**F-38340 VOREPPE (FR)**
• **RICOUL, Florence**
**F-38950 QUAIX-EN-CHARTREUSE (FR)**

(74) Mandataire: **Poulin, Gérard**
**Brevatome**
**3, rue du Docteur Lancereaux**
**75008 Paris (FR)**

(56) Documents cités:
WO-A-03/016550          WO-A-03/083127
US-A1- 2002 102 567     US-B1- 6 423 535

• **SCHUCHHARDT J ET AL: "NORMALIZATION STRATEGIES FOR CDNA MICROARRAYS" NUCLEIC ACIDS RESEARCH, OXFORD UNIVERSITY PRESS, SURREY, GB, vol. 28, no. 10, 15 mai 2000 (2000-05-15), pages E47I-E47V, XP000982625 ISSN: 0305-1048**

**EP 1 706 509 B1**

**Description**

**DOMAINE TECHNIQUE**

La présente invention se rapporte à une puce d'analyse comportant une gamme étalon, à un procédé d'analyse d'un échantillon utilisant ladite puce, et à une trousse de diagnostic ou d'analyse comprenant ladite puce.

La présente invention est adaptée à l'analyse qualitative et surtout quantitative de tout analyte présent dans un échantillon.

La présente invention se situe donc notamment dans le domaine des puces chimiques ou biologiques, par exemple à ADN, à protéines, à anticorps, à antigènes, à cellules, à récepteurs, ou à d'autres ligands connus de l'homme du métier et capables de capturer et fixer spécifiquement un ou plusieurs analytes.

Habituellement, les applications utilisant des puces à ADN sont scindées en deux grandes catégories : l'expression de gènes (i) et le génotypage (ii). (i) L'expression de gène consiste à utiliser une ou des puce(s) à ADN pour étudier la variation de tout ou partie du transcriptome d'une cellule selon certains paramètres biologiques ou physiologiques : évolution d'une cellule au cours du développement d'un organisme, de la cancérisation d'un tissu, etc. Il est de plus en plus clair que de tels profils d'expression peuvent aussi servir de marqueur diagnostique pour les maladies multifactorielles, notamment en cancérologie. (ii) Dans les applications de type génotypage, la réponse demandée à la puce à ADN est en général de type oui/non, qualitative. En fait, on veut savoir si une ou plusieurs séquence(s) particulière(s) est (sont) présente(s) dans l'échantillon. Quand un grand besoin de spécificité est requis, par exemple dans le cas de la détection de mutations, plusieurs oligonucléotides qui ne diffèrent que par une ou deux bases, sont placés sur la puce, et en général, on conclut à la présence de la séquence correspondant au signal, par exemple de fluorescence, le plus fort.

La présente invention est adaptée à ces deux grandes catégories d'applications et résout avantageusement les nombreux problèmes de l'art antérieur qui sont présentés ci-dessous, en particulier dans l'élaboration de tels études et profils.

Grâce à sa gamme étalon, la puce de la présente invention permet de convertir des signaux de détection et/ou d'analyse d'une puce, par exemple d'une puce biologique, en une unité absolue, reproductible, stable, et comparable à d'autres résultats de mesure obtenus à partir de la même conversion.

**ETAT DE LA TECHNIQUE ANTERIEURE**

Pour la quantification de variations d'expression de gènes, le biologiste utilise presque toujours deux échantillons qui sont hybridés en même temps sur une puce à ADN : un échantillon de référence, comprenant une certaine masse d'ARN ou ADNc marqué avec un premier fluorochrome, par exemple la fluorescéine (lue dans le vert), et un échantillon d'intérêt où la mesure doit être effectuée, comprenant la même masse d'ARN ou ADNc marqué avec un deuxième fluorochrome, par exemple Cy3 ou Cy5 (lu dans l'orange ou le rouge). Après hybridation, la puce à ADN est lue aux deux longueurs d'onde, et c'est le rapport entre les deux intensités des signaux émis qui sert de signal.

Il s'agit donc d'une mesure relative avec référence interne dans chaque expérience. Cette méthode fonctionne bien, mais est assez laborieuse à mettre en oeuvre, et certains pièges existent, comme des biais de marquage selon les séquences, ou une dérive de lecture différente selon la longueur d'onde.

Une autre technique de quantification existe pour les puces d'expression de gènes, mise au point par la société Affymetrix. Dans cette méthode, chaque séquence mise sur la puce est représentée par un ensemble d'oligonucléotides, et chaque oligonucléotide existe sous la forme de deux oligonucléotides, l'un parfaitement complémentaire à la séquence visée, l'autre comportant une mutation ponctuelle (« mismatch »). L'échantillon d'intérêt est alors hybridé sur la puce, une seule couleur est utilisée. La différence complémentaire-mismatch est alors utilisée comme signal primaire, ces signaux étant moyennés sur l'ensemble des oligonucléotides représentant un gène. Par ailleurs, on dispose un "gène étalon" sur la puce selon la même technique ; ce gène étalon, pris dans les gènes dits de ménage de l'organisme, est supposé rester d'expression constante quelles que soient les conditions physiologiques de la cellule. On divise alors le "signal primaire" de chaque gène avec celui du gène de référence, pour s'affranchir par exemple des variations de brillance de la fluorescence par l'environnement. On peut alors comparer différentes expériences entre elles.

Cette technique présente l'avantage d'être monochrome, donc n'a pas les inconvénients cités plus haut avec les deux marqueurs. Cependant, elle utilise forcément des oligonucléotides courts (20 à 25 bases), et d'autres pièges existent donc, car certains gènes existent sous différentes formes (épissage alternatif). Cette méthode nécessite donc d'avantages de sites sur la puce à ADN, et plus de connaissances sur les différentes formes des ARNs des gènes représentés sur la puce qu'avec les autres méthodes de l'art antérieur. De plus, les résultats varient selon le choix du gène étalon, même quand celui-ci est choisi précautionneusement. La comparaison des résultats n'est donc pas toujours possible.

Les méthodes précitées, moyennant certains contrôles et certaines répétitions des expériences, peuvent être relativement fiables, et, fournissent donc un moyen d'étude utilisable par les biologistes. Cependant, il faut noter que les mesures sont relatives, puisque le signal est un nombre sans dimension résultant du rapport entre deux mesures aux unités en général arbitraires (unités arbitraires de fluorescence). Il n'est donc pas possible de comparer des expériences faites avec des échantillons de référence différents par exemple, sans expérience complémentaire. De plus, les mesures étant en unité arbitraire, il est également assez difficile de savoir d'où viennent les problèmes en cas d'intensités faibles : il est difficile de savoir s'il s'agit d'un problème instrumental, d'un problème lié à la puce elle-même, à l'échantillon, au protocole opératoire, ou au résultat de l'expérience.

Dans les applications de type génotypage, la réponse demandée à la puce à ADN est en général de type oui/non, qualitative, comme expliqué ci-dessus. Là encore, sur les puces de l'art antérieur, les résultats sont relatifs, puisqu'on compare entre elles les intensités de différents oligonucléotides recherchés sur une même puce.

Toutefois, pour certaines applications, comme la détection de mélanges de plusieurs séquences, il serait avantageux de pouvoir comparer les signaux entre plusieurs expériences, ce qui est aujourd'hui impossible avec les puces actuellement disponibles ; il faut en permanence refaire une ou plusieurs expériences pour avoir des mesures relatives permettant de conclure, ce qui est long et coûteux.

Par ailleurs, en diagnostic de routine, le problème de la quantification des résultats peut être résolu par une courbe de calibrage. Un échantillon de référence est dilué de façon prédéterminée à différentes concentrations dans un diluant approprié, et le test biologique est réalisé pour chaque dilution en dehors de la puce. On obtient ainsi une courbe étalon. Quand on réalise un test biologique réel, le résultat en unité arbitraire, est reporté sur la courbe étalon, et on en déduit la concentration en analyte de l'échantillon. Cependant, il est important que le test soit réalisé avec exactement les mêmes éléments que ceux utilisés pour l'établissement de la courbe étalon. En outre, dans les tests avec révélation enzymatique pour la lecture, par exemple les tests ELISA, ELOSA, etc., il faut en plus une courbe étalon pour chaque lot de fabrication des tests. De plus, des points supplémentaires de calibrage permettant de recaler la courbe étalon pour chaque utilisateur doivent être réalisés pour corriger des variations d'une machine à une autre, d'une température à une autre, d'un laboratoire à un autre, d'un vieillissement possible des réactifs, etc. Ces tests supplémentaires de calibrage doivent être constamment renouvelés par les utilisateurs, par exemple, toutes les semaines.

Cette méthode de quantification, pourtant usuelle, est donc complexe et coûteuse. De plus, elle ne s'applique pas bien aux puces à ADN ; en effet, il faudrait utiliser une puce à ADN pour chaque point de la gamme étalon, et répéter constamment l'expérience (à chaque changement de lot de marqueur, ou à période régulière, par exemple tous les mois, pour surveiller une dérive éventuelle de l'instrumentation), ce qui est trop coûteux pour les laboratoires.

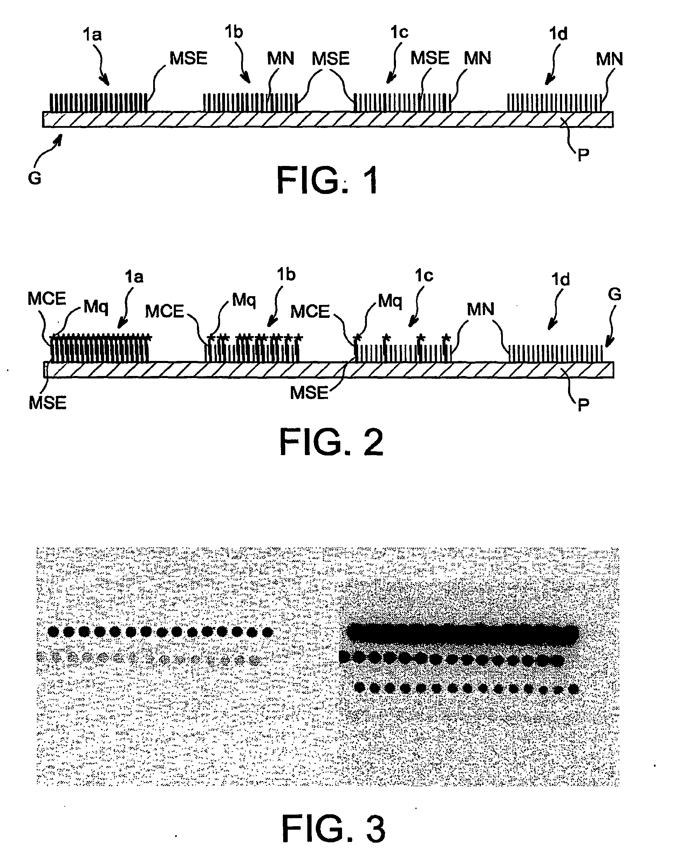

Schuchhardt et al., (2000) [17] décrit une puce d'analyse d'au moins un analyte présent dans un échantillon liquide, ladite puce comprenant a) plusieurs sites d'analyse disposés sur la puce de façon à permettre la reconnaissance et la fixation spécifique de l'analyte, et une gamme étalon (Materials and methods: Array preparation). La gamme étalon est constituée de plusieurs sites étalons comportant au moins une molécule sonde étalon permettant de reconnaître et de se fixer spécifiquement à une molécule cible étalon déterminée (Figure 1).

Il existe donc un réel besoin d'une puce et d'un procédé d'analyse qui permettent de résoudre les nombreux problèmes précités de l'art antérieur, et notamment qui soient fiables, précis, reproductibles ; qui s'appliquent aux différentes puces à ADN connues et futures ; qui permettent de comparer les signaux entre plusieurs expériences ; qui permettent d'éviter d'utiliser une puce à ADN pour chaque point de gamme étalon ; et qui permettent d'éviter de répéter constamment l'expérience d'étalonnage (à chaque changement de lot de marqueur, ou à période régulière, par exemple tous les mois, pour surveiller une dérive éventuelle de l'instrumentation), afin de diminuer la complexité et les coûts analyses sur puce, par exemple sur puce biologique, pour les laboratoires, dans l'industrie et dans la recherche.

Dans la description suivante, les références entre crochets [ ] renvoient à la liste de référence donnée après les exemples de réalisation de l'invention décrits ci-dessous.

## EXPOSÉ DE L'INVENTION

La présente invention répond précisément à ce besoin et résout les nombreux problèmes précités de l'art antérieur, et d'autres encore, en fournissant notamment une puce d'analyse, ainsi qu'un procédé d'analyse utilisant cette puce tels qu'ils sont définis ci-dessous.

La puce d'analyse de la présente invention est une puce d'analyse d'au moins un analyte présent dans un échantillon liquide, ladite puce comprenant :

a) au moins un site d'analyse dudit, au moins un, analyte, ledit site d'analyse étant disposé sur la puce permettant la reconnaissance et la fixation spécifique de l'analyte ; et

b) une gamme étalon (G), ladite gamme étalon étant constituée de plusieurs sites étalons disposés chacun sur ladite puce de manière déterminée et indépendants les uns des autres, chaque site étalon de cette gamme comportant, fixé(s) à sa surface et en proportion déterminée P différente pour chaque site par rapport aux autres sites étalons de ladite gamme : (i) au moins une molécule sonde étalon (MSE) permettant de reconnaître et de se fixer spécifiquement à une molécule cible étalon (MCE) déterminée, et (ii) au moins une molécule neutre (MN) incapable de reconnaître et de se fixer à ladite molécule cible étalon, la molécule sonde étalon et la molécule neutre étant toutes les deux incapables de reconnaître et de fixer ledit ou lesdits analyte(s) ; avec

$$P = \frac{\text{nombre de MSE}}{\text{nombre de MSE} + \text{nombre de MN}}$$ et $0 \leq P \leq 1$, la somme de nombre d'MSE + nombre d'MN étant constante d'un site étalon à l'autre.

Le procédé d'analyse de la présente invention est un procédé d'analyse *in vitro* d'au moins un analyte susceptible d'être présent dans un échantillon liquide comprenant les étapes suivantes :

(α) optionnellement, fixation sur l'analyte d'un premier moyen de détection ;
(β) addition dans l'échantillon à analyser, comprenant l'analyte optionnellement marqué, d'une molécule cible étalon (MCE) sur laquelle un deuxième moyen de détection, identique ou différent du premier moyen de détection, a optionnellement été fixé, ladite MCE permettant de reconnaître et de se fixer spécifiquement à la molécule sonde étalon (MSE) d'une puce d'analyse selon l'invention, ladite MCE étant ajoutée audit échantillon en une quantité suffisante pour saturer les MSE de la gamme étalon (G) de ladite puce, créant ainsi une gamme étalon fonction de la proportion déterminée P différente pour chaque site étalon ;
(γ) mise en contact de l'échantillon à analyser comprenant la MCE avec ladite puce d'analyse comprenant au moins un site d'analyse dudit au moins un analyte dans des conditions physicochimiques telles que : d'une part l'analyte à analyser, s'il est présent, se fixe sur son site d'analyse sur la puce ; et d'autre part la MCE reconnaisse spécifiquement la MSE et se fixe à celle-ci sur les différents sites étalon de la gamme étalon de la puce ;
(δ) détermination d'un signal étalon émis, le cas échéant grâce au deuxième moyen de détection, par chaque site étalon de la gamme étalon, ledit signal étant fonction de la quantité de molécule cible étalon fixée sur celui-ci ; et
(ε) détermination d'un signal d'analyse émis, le cas échéant grâce au premier moyen de détection, par ledit, au moins un, site d'analyse et fonction de la quantité d'analyte fixé par le site d'analyse, et rapprochement aux signaux déterminés à l'étape (δ) pour exprimer ce signal d'analyse en fonction de P.

Par « analyte », on entend tout ou partie de corpuscule ou molécule que l'on désire analyser, c'est à dire détecter et/ou quantifier, par exemple un microorganisme, une bactérie, un champignon, un virus, une cellule eucaryote, une cellule telle qu'une cellule tumorale, un composé ou molécule chimique, une molécule telle qu'un peptide, une protéine, une glycoprotéine, une lipoprotéine, un enzyme, un polysaccharide, un lipide, un glycolipide, un lipopolysaccharide, un acide ribonucléique (ARN), un acide désoxyribonucléique (ADN), une hormone, un antigène, un anticorps, un facteur de croissance, un haptène, etc.

L'échantillon peut être une solution, un extrait cellulaire, un prélèvement effectué sur un organisme animal ou végétal. Cet échantillon peut être dilué, si nécessaire, pour son utilisation dans la présente invention. L'homme du métier connaît la manipulation de tels échantillons et leur mise en solution et/ou leur dilution, leur purification et/ou leur concentration, pour leur analyse sur une puce d'analyse, par exemple une puce biologique ou chimique. Ces mêmes manipulations s'appliquent pour la mise en oeuvre de la présente invention.

La puce de la présente invention comprend, en combinaison, d'une part au moins un site d'analyse d'au moins un analyte à analyser et d'autre part une gamme étalon, tous deux disposés à la surface d'une même puce conformément à la présente invention.

La puce de la présente invention est constituée de la même manière que les puces d'analyse de l'art antérieur, hormis qu'elle comprend en plus une gamme étalon au sens de la présente invention. Les étapes de fabrication de la gamme étalon s'ajoutent donc à celles de fabrication de la puce. Des puces à ADN utilisables pour la mise en oeuvre de la présente invention et leur(s) procédé(s) de fabrication sont décrits par exemple dans le document [1] de la liste de référence. Des puces à protéines utilisables pour la mise en oeuvre de la présente invention et leur(s) procédé(s) de fabrication sont décrits par exemple dans les documents [2] et [3] de la liste de référence.

La puce de la présente invention présente donc un spectre très large d'utilisations en tant qu'outil d'analyse. Aussi, par « analyse » on entend « analyse qualitative » d'un échantillon, c'est à dire détection d'analyte(s) présent(s) dans un échantillon et/ou « analyse quantitative », c'est à dire dosage d'analyte(s) présent(s) dans un échantillon. Des exemples sont donnés ci-dessous.

Selon l'invention, les « sites » (appelés « spots » en anglais) comportent les moyens permettant de reconnaître et de fixer exclusivement et spécifiquement soit « l'analyte » (site d'analyse) soit « la molécule cible étalon » (site de la gamme étalon).

Pour le « site d'analyse », ces moyens comprennent des molécules sondes spécifiques dudit analyte. La molécule sonde spécifique peut être par exemple une molécule d'ADN ou d'ARN permettant une reconnaissance et une fixation d'un analyte complémentaire (ADN ou ARN) par hybridation ; d'un antigène ou d'un anticorps reconnaissant et fixant l'analyte par une interaction de type antigène/anticorps ; d'une protéine reconnaissant et fixant l'analyte par une interaction de type protéine/protéine ; d'un enzyme ou d'un substrat reconnaissant et fixant l'analyte par une interaction de type enzyme/substrat ; etc.

Par exemple, lorsque l'analyte est un acide nucléique, le, au moins un, site d'analyse est un site fonctionnalisé par un acide nucléique complémentaire à celui-ci. Par exemple aussi, lorsque l'analyte est un anticorps ou un antigène, le, au moins un, site d'analyse est un site fonctionnalisé respectivement par un antigène ou un anticorps.

Les procédés de fabrication de ces sites d'analyse et leur fonctionnalisation sont connus de l'homme du métier, et sont exposés par exemple dans les documents précités. La fonctionnalisation des sites d'analyse de la puce peut être effectuée par exemple au moyen d'un robot dispenseur de gouttes d'une solution de fonctionnalisation, par exemple un robot de type Packard Instrument ou GeSim (marques de commerce).

La puce de la présente invention peut comprendre plusieurs sites d'analyse, identiques ou différents, de la même manière que les puces d'analyse actuellement utilisées en laboratoire.

La gamme étalon de la présente invention est constituée de sites étalons, qui sont également des sites de reconnaissance et de fixation d'une cible, mais ces sites étalons sont caractérisés en ce qu'ils ne peuvent pas reconnaître et fixer le ou les analytes à analyser, mais uniquement une molécule cible étalon déterminée. Les sites étalons de cette gamme étalon sont tels que défini ci-dessus dans les points (i) et (ii) du procédé de l'invention.

Selon l'invention la molécule sonde étalon, la molécule cible étalon, et la molécule neutre sont choisies ensembles et en fonction de l'analyte auquel est destinée la puce de la présente invention, afin qu'elles interagissent le moins possible avec l'analyte.

Selon l'invention, de préférence, la molécule cible étalon est de même nature que l'analyte. Elle peut également être de nature différente. L'essentiel est que la molécule cible étalon ne soit ni reconnue ni fixée par ledit, au moins un, site d'analyse

Selon l'invention, la molécule sonde étalon est choisie pour reconnaître et fixer spécifiquement ladite molécule cible étalon. De préférence, cette molécule sonde étalon est de même nature que la molécule sonde spécifique de l'analyte. Elle peut également être de nature différente. L'essentiel est que la molécule sonde étalon ne reconnaisse pas et ne fixe pas l'analyte.

Par exemple, selon l'invention, la molécule cible étalon et la molécule sonde étalon peuvent être par exemple des oligonucléotides (ADN ou ARN) complémentaires pour une reconnaissance et une fixation de la molécule cible étalon par hybridation sur les sites de la gamme étalon comportant la molécule sonde ; par exemple un antigène et son anticorps spécifique permettant une reconnaissance et une fixation de la molécule cible étalon par une interaction de type antigène/anticorps sur les sites de la gamme étalon comportant la molécule sonde; par exemple des protéines permettant une reconnaissance et une fixation de la cible étalon par une interaction de type protéine/protéine sur les sites de la gamme étalon comportant la molécule sonde ; etc.

Selon l'invention, le au moins un analyte et la molécule cible étalon sont de préférence des oligonucléotides ou des anticorps. La fonctionnalisation des différents sites (analyse et gamme étalon) sur une puce conforme à la présente invention utilisant des oligonucléotides ou des anticorps ou antigènes sondes et/ou neutre peut être mise en oeuvre en utilisant les techniques de fonctionnalisation que l'homme du métier maîtrise parfaitement dans le domaine des puces à oligonucléotides ou anticorps ou antigènes. Des techniques utilisables sont décrites par exemple dans les documents précités.

Selon l'invention, la molécule neutre de la gamme étalon, appelée aussi molécule « non spécifique », est choisie de manière à ce qu'elle ne reconnaisse pas ni ne fixe la molécule cible étalon et le ou les analyte(s). De préférence, selon l'invention, la molécule neutre est de même nature que la molécule sonde étalon. En effet, cela permet de tenir compte des interactions non spécifiques qui pourraient interférer dans l'analyse, au cours de l'utilisation de la puce de la présente invention, par exemple entre la molécule neutre et le ou les analyte(s) d'une part et la molécule cible étalon d'autre part, et d'obtenir une gamme étalon fiable. Dans les exemples précédents, la molécule neutre de la gamme étalon peut donc être respectivement un ADN ou un ARN, un antigène ou un anticorps, une protéine, un enzyme ou un substrat. La molécule neutre étalon est également fixée sur les sites de la gamme étalon suivant les techniques de fonctionnalisation des puces connues de l'homme du métier, par exemple celles décrites dans les documents précités.

Selon l'invention, de préférence la molécule sonde étalon, la molécule cible étalon et la molécule neutre sont des oligonucléotides. La fabrication de séquences oligonucléotidiques, par exemple artificielles, répondant aux définitions des molécules sonde et cible étalon et de la molécule neutre de la présente invention, et la fonctionnalisation de sites sur une puce en utilisant ces oligonucléotides sonde et neutre pour la fabrication de la gamme étalon est en effet aisée et utilise les techniques que l'homme du métier maîtrise parfaitement dans le domaine des puces à oligonucléotides.

Cas des systèmes in situ : certains procédés de fabrication de puces à ADN utilisent une synthèse in situ des oligonucléotides (par exemple les procédés de fabrication des puces GeneChip de la société Affymetric (marque déposée), ou les puces de la société Agilent). Dans ces procédés, chaque oligonucléotide est synthétisé in situ sur la puce. L'adressage spatial des monomères à coupler est fait soit au niveau de la déprotection dans un cycle classique de synthèse d'oligonucléotides (photo-déprotection pour Affymetrix) ; soit au niveau du couplage des monomères (« spotting » pour Agilent). Une telle technique de synthèse in situ permet de fabriquer les sites d'analyse de la puce de la présente invention, et, en ajoutant autant de cycles de synthèse qu'il y a de points de la gamme étalon, et dans ces cycles de synthèse, en couplant directement le mélange oligonucléotide sonde étalon/oligonucléotide neutre (non spécifique) à l'endroit qui convient sur le support formant la puce (au lieu de ne coupler qu'un monomère), on fabrique une gamme étalon selon l'invention.

Selon l'invention, la gamme étalon est constituée de plusieurs sites étalons disposés sur ladite puce de manière déterminée et indépendants les uns des autres. En effet, pour que la gamme étalon puisse être utilisable il est nécessaire que chaque site étalon soit distinct des autres sites étalons afin que les différents sites de cette gamme fournissent des signaux indépendants et exploitables pour l'analyse lors de l'utilisation de la puce.

Chaque site de la gamme étalon se distingue des autres sites de cette gamme par sa proportion déterminée P telle que définie ci-dessus : la quantité de molécules sondes étalons par rapport aux molécules neutres est donc connue pour chaque site étalon. Cette proportion déterminée P est obtenue sur chaque site étalon en fonctionnalisant chaque site, indépendamment des autres, au moyen d'une solution de concentration déterminée en molécule sonde étalon et/ou en molécule neutre. Selon l'invention cette proportion P peut aller de 0, dans le cas où le site étalon ne comporte que la molécule neutre, à 1, dans le cas où le site étalon ne comporte que la molécule sonde étalon. L'essentiel est que la quantité totale de molécule sonde étalon et de molécule neutre (MSE + MN) soit égale d'un site à l'autre, afin que la gamme étalon soit précise.

De préférence, dans l'exemple où les molécules sonde étalon et neutre sont des oligonucléotides, ils sont choisis de telle façon que leur efficacité de greffage sur les sites de la gamme étalon lors de la fabrication de la puce soit égale. Ainsi, après greffage, la proportion d'oligonucléotide étalon est égale à celle de la dilution de départ. Ceci permet de passer avantageusement de l'unité « % de dilution » à l'unité « % des oligonucléotides étalons greffés » au cours d'une analyse utilisant la puce de la présente invention.

Dans l'exemple de puces à oligonucléotides conformes à la présente invention, par opposition aux puces à ADNc ou à produit de PCR, de préférence, la quantité globale « oligonucléotides sondes étalons + oligonucléotides neutres non spécifiques » est équivalente à la quantité de sondes oligonucléotidiques utilisées pour chaque site d'analyse pour la fabrication de la puce. On utilisera également, de préférence, des oligonucléotides de même longueur. Ainsi, l'efficacité de greffage étant peu dépendante de la séquence, pour chaque site, il est possible d'exprimer le signal en « pourcentage de sites hybridés », 100% correspondant à la saturation de la surface.

De préférence, les sites de la gamme étalon sont disposés sur la puce de manière linéaire et sont ordonnés en fonction de la proportion P de chaque site, par exemple du site le plus concentré au site le moins concentré en molécule sonde étalon. En effet cela donne une échelle d'étalonnage plus facilement exploitable lors de l'utilisation de la puce. L'augmentation de la proportion P d'un site étalon à un autre voisin de celui-ci dans une telle gamme peut être linéaire, par exemple, en pourcentage de molécule sonde étalon sur différents sites successifs, de 0% ; 20% ; 40% ; 60% ; 80% et 100%, ou non linéaire, par exemple 0%, 5%, 10%, 20%, 50%, 100% ou encore 0% ; 0,1% ; 1% ; 10% et 100%. Il n'y a pas d'autre restriction pour la mise en oeuvre de la présente invention que celles de pouvoir attribuer clairement à un signal donné d'un site de la gamme étalon une proportion de molécule sonde étalon déterminée.

Le nombre de sites étalons de la gamme étalon est déterminé en fonction de la précision d'analyse recherchée et de la gamme dynamique du test lors de l'utilisation de la puce. Par exemple trois sites étalons présentant par exemple successivement 0%, 50% et 100% de molécule sonde étalon ne fournissent que trois résultats d'étalonnage sur la base desquelles les résultats d'analyse de l'analyte devront se fonder, alors que cinq sites étalons présentant par exemple successivement 0%, 20%, 40%, 60%, 80% et 100% de molécule sonde étalon fournissent six résultats d'étalonnage permettant une lecture plus précise des résultats d'analyse. A titre d'exemple, la gamme étalon peut comprendre de 1 à 50 sites étalons, par exemple de 2 à 20. L'homme du métier déterminera aisément, suivant la précision d'analyse recherchée et la gamme dynamique des concentrations, le nombre de sites étalons différents requis et leur agencement ou disposition sur la puce.

La figure 1 annexée représente schématiquement, en coupe, une gamme étalon (G) sur une puce (P) conforme à la présente invention. Sur cette figure, sont représentés différents sites étalons (la, 1b, 1c et 1d), sur lesquels sont fixées les molécules sondes étalons (MSE) (en traits gras) et les molécules neutres (MN) (en traits fins). Les sites d'analyse ne sont pas représentés. Le site le plus à gauche ne comporte que MSE (100%), et le site le plus à droite que MN (0% MSE). Les sites intermédiaires comportent des proportions intermédiaires de MSE. La somme MSE + MN est constante d'un site à l'autre.

Selon l'invention, plusieurs gammes étalons, identiques ou différentes, peuvent être disposées sur une même puce. L'utilisation de plusieurs gammes étalons, bien que n'étant pas obligatoire, permet dans certains cas d'améliorer la précision des analyses effectuées. Cela peut être le cas par exemple lorsque l'échantillon comprend plusieurs analytes à analyser simultanément sur la puce, par un oligonucléotide et un anticorps. L'homme du métier déterminera aisément, suivant l'analyse à laquelle est destinée la puce, la mise en oeuvre la plus appropriée.

La présente invention révèle toute son importance dans l'utilisation de la puce comportant cette gamme étalon pour l'analyse d'un échantillon comprenant ou susceptible de comprendre un ou plusieurs analytes à analyser, notamment dans le procédé de l'invention.

Dans le procédé de l'invention, l'analyte et la molécule cible étalon sont de préférence de même nature, sans être limité à ce mode de réalisation. De préférence aussi, l'analyte et la molécule cible étalon sont des oligonucléotides ou des anticorps, sans être limité à ce mode de réalisation. Dans un mode de réalisation particulièrement avantageux de mise en oeuvre de l'invention, la molécule sonde étalon, la molécule cible étalon et la molécule neutre sont des oligonucléotides pour les mêmes raisons que celles exposées ci-dessus.

De la même manière, et pour les mêmes raisons que celles exposées ci-dessus, le, au moins un, analyte est avantageusement un acide nucléique, et le site d'analyse dudit analyte sur la puce est un site fonctionnalisé par un acide nucléique complémentaire à celui-ci. L'analyte peut également être un antigène ou un anticorps, et le site d'analyse dudit analyte sur la puce un site fonctionnalisé respectivement par un anticorps ou un antigène reconnaissant et fixant celui-ci.

L'étape ($\alpha$) du procédé de l'invention est optionnelle. Dans cette étape, un premier moyen de détection peut être fixé sur l'analyte. Il peut s'agir de tout moyen connu de l'homme du métier utile pour mettre en évidence un analyte fixé sur une molécule sonde spécifique (ensemble sonde/analyte) fixée sur une puce. Ce moyen peut impliquer par exemple un premier marqueur, qui peut être fixé soit sur la molécule sonde spécifique, soit sur l'analyte. Ce moyen peut également être une autre molécule pour un marquage indirect, par exemple une biotine, suivant la technique de marquage utilisée. Les marqueurs et les techniques de marquage utilisables pour la mise en oeuvre de la présente invention sont sans limitation ceux connus de l'homme du métier dans le domaine des puces d'analyse, par exemple des puces biologiques, par exemple des puces à ADN ou ARN, des puces à anticorps/antigènes ou des puces utilisant une interaction enzyme/substrat.

Les marqueurs utilisables peuvent être choisis par exemple dans le groupe constitué des marqueurs fluorescents; des marqueurs radioactifs ; des particules de latex, standards, colorées ou fluorescentes ; des cristaux photoniques (appelés aussi « quantum dots ») ; d'or colloïdal ; et des marqueurs enzymatiques. Parmi les marqueurs fluorescents, on peut citer la fluorescéine, le Cy3, le Cy5 et la rhodamine. Parmi les marqueurs radioactifs, on peut citer par exemple $P^{32}$, $P^{33}$, $S^{35}$, $I^{125}$, $H^3$. Parmi les marqueurs enzymatiques, on peut citer la phosphatase alcaline (PAL), la peroxydase de raifort (HRP ou « horse radisch peroxydase »), la $\beta$-galactosidase, l'acétylcholestérase. Pour ces enzymes, on trouve des marqueurs colorés, fluorescents ou luminescents. Dans ce cas, ce sont des molécules transformées par l'enzyme, et qui, après transformation, deviennent colorées (ou changements de longueur d'onde d'absorption), fluorescentes, luminescentes ou redox. Les marqueurs enzymatiques qui sont utilisés en routine dans les immunoanalyses en diagnostic peuvent également être utilisés. Ces marqueurs fluorescents ou enzymatiques, et d'autres encore utilisables dans la présente invention, sont disponibles par exemple à partir des catalogues des sociétés Sigma/Aldrich, Molecular Probes, Amersham Pharmacia Biotech., Stratagene, etc. Les documents [4] et [5] de la liste de référence décrivent des procédés d'utilisation de ces marqueurs utilisables pour la mise en oeuvre de la présente invention. Des marqueurs sous forme de particules utilisables dans la présente invention sont disponibles chez plusieurs fournisseurs, par exemple Molecular Probe, Miltenyi, Estapor/Merck, Polymer Solutions, etc. Le document [6] de la liste de références décrit des procédés mettant en oeuvre ces marqueurs sous forme de particules, latex ou colloïde utilisables pour la mise en oeuvre de la présente invention. Pour l'utilisation de cristaux photoniques on pourra utiliser par exemple le procédé décrit dans le document [7] de la liste de référence.

Pour certains marqueurs, en particulier les marqueurs enzymatiques, le produit détectable peut s'éloigner du site (« spot ») où il a été produit, par diffusion moléculaire, et contaminer des sites voisins. Ceci peut être gênant pour l'utilisation à la fois de la gamme étalon et des signaux émis par les sites de reconnaissance du ou des analyte(s). C'est pourquoi les inventeurs préfèrent des marqueurs luminescents, car la diffusion est limitée : une fois qu'il a émis un photon, le produit enzymatique ne peut plus en émettre. Pour éviter le phénomène de diffusion, il est également possible d'utiliser des substrats enzymatiques dits précipitants : le produit enzymatique est insoluble, et précipite sur place, c'est-à-dire sur le site. Par exemple, on peut utiliser la tétraméthylbenzidine (TMB) pour l'enzyme HRP, et l'association Nitro Bleu de Tetrazolium avec le Phosphate 5-Bromo-4-Chloro-Indolyl (NBT + BCIP)) pour la phosphatase alcaline.

Selon l'invention, afin de parer à ces phénomènes de diffusion qui peuvent gêner la lecture de la puce avec certains marqueurs, mais aussi, de manière générale, afin de parer à tout phénomène de diffusion entre sites voisins, les inventeurs préconisent l'utilisation de puces sur lesquelles les sites (de reconnaissance et de fixation de l'analyte et/ou de la gamme étalon) sont pourvus de moyens qui permettent la formation circonscrite de gouttes de l'échantillon obtenu à l'étape ($\beta$) exclusivement sur chacun des sites, sans que ces gouttes puissent diffuser entre elles. Ces moyens peuvent être par exemple sous la forme d'une bordure, par exemple de résine, entourant chaque site. Ces moyens peuvent également se présenter sous la forme d'un anneau entourant chaque site et présentant une mouillabilité pour l'échantillon très supérieure au reste de la surface de la puce, afin qu'une goutte d'échantillon soit retenue par chaque anneau exclusivement sur chaque site. Cet anneau peut être par exemple un anneau de silicium noir obtenu par micro gravure sur une surface silice ou une électrode de capture d'une goutte d'échantillon par électro-mouillage. Quels que soient ces moyens permettant la formation de gouttes, la surface de la puce est de préférence non mouillante vis-à-vis de l'échantillon. La puce de la présente invention peut donc avantageusement être pourvue de ces moyens afin d'augmenter la qualité ou la fiabilité des mesures obtenues.

Dans l'étape (β), on ajoute à l'échantillon, tel qu'il est défini ci-dessus, la molécule cible étalon sur laquelle est optionnellement fixé un deuxième moyen de détection identique ou différent du premier moyen de détection. Ce deuxième moyen peut impliquer par exemple un deuxième marqueur, qui peut être fixé sur la molécule cible étalon ou sur la molécule sonde étalon. Il peut également impliquer une molécule différente d'un marqueur mais qui intervient dans un procédé de marquage indirect (par exemple biotine). Ce marqueur peut être choisi par exemple parmi ceux précités. Le deuxième moyen de détection est de préférence, mais non obligatoirement, identique au premier moyen de détection. L'essentiel, est que la fixation de la molécule cible étalon sur la molécule sonde étalon de la gamme étalon puisse être détectée sur la puce par un signal détectable.

Selon l'invention, seul l'analyte peut être marqué, ou alors seule la molécule cible étalon, ou les deux, ou aucun des deux. En effet, certaines techniques de détection permettent de détecter une interaction moléculaire (fixation sonde/cible sur une puce) sans marqueur. Des exemples sont donnés ci-dessous.

La molécule cible étalon sur laquelle est optionnellement fixé le deuxième moyen de détection est ajoutée à l'échantillon en quantité suffisante pour que le maximum de molécules sondes des sites de la gamme étalon reconnaissent et fixent la molécule cible dans les conditions expérimentales utilisées pour la mise en contact (étape γ)) de l'échantillon avec la puce. L'addition à l'échantillon peut se faire par mélange, de préférence homogène, avec l'échantillon.

Dans l'étape (γ), on met en contact le mélange de l'étape (β) avec la puce de la présente invention, dans les conditions physicochimiques définies ci-dessus. Ces conditions sont connues de l'homme du métier dans le domaine des puces d'analyse, par exemple des puces biologiques, par exemple des puces à ADN, à ARN, à anticorps/antigène, à protéines, etc. Il s'agit, par exemple pour les puces précitées, des conditions de pH, de température et de force ionique permettant la reconnaissance et la fixation du ou des analyte(s) (ADN, ARN, anticorps, antigène, protéine, etc.) sur son ou leur(s) site(s) de reconnaissance, et de la molécule cible étalon (ADN, ARN, anticorps, antigène, protéine, etc.) sur les sites de la gamme étalon comportant la molécule sonde étalon. L'avantage de la présente invention est que la reconnaissance et la fixation de l'analyte par le, au moins un, site d'analyse et la formation de la gamme étalon par la fixation de la molécule sonde étalon sur les sites étalon sont effectuées simultanément et dans les mêmes conditions opératoires. L'impact est évident : les signaux émis par la gamme étalon ont été obtenues dans les mêmes conditions opératoires que celles utilisées pour la fixation de l'analyte. Les résultats d'analyse sont donc plus fiables que ceux obtenus par les procédés de l'art antérieur.

De manière générale, la mise en contact des sites d'analyse et des sites de la gamme étalon avec l'échantillon peut se faire par les moyens habituels de la technique des puces d'analyse utilisés pour la répartition d'un échantillon sur des zones fonctionnalisées.

Sur une puce comprenant des moyens qui permettent la formation circonscrite de gouttes de l'échantillon sur les sites de la puce, cette mise en contact peut se faire très facilement en couvrant les sites avec l'échantillon, puis en retirant l'échantillon de manière à ne laisser que des gouttes d'échantillon capturées par ces moyens sur les sites d'analyse et les sites de la gamme étalon de la puce.

La figure 2 annexée représente schématiquement, en coupe, la gamme étalon (G) de la figure 1 après l'étape de mise en contact. Sur cette figure, sont représentés les molécules cibles étalons (MCE) fixées sur les molécules sondes étalons (MSE). Les MCE sont marquées au moyen d'un marqueur (Mq). Il est clair que le site le plus à gauche (100% MSE) donnera le signal du marqueur le plus fort, et le site le plus à droite (0% MSE) ne donnera pas de signal. Des signaux intermédiaires apparaîtront sur les sites du milieu.

Comme pour l'utilisation des puces d'analyse connues de l'homme du métier cette étape (γ) peut être suivie d'étape(s) de lavage et de rinçage dans des conditions physicochimiques qui ne détruisent pas la fixation de l'analyte sur son site d'analyse, ainsi que la fixation de la molécule cible étalon sur la molécule sonde étalon. Ces étapes de lavage et de rinçage sont connues de l'homme du métier et peuvent être trouvées dans les documents précités. Elles permettent de retirer les excès de réactifs et molécules non fixées sur la puce.

Dans l'étape (δ) du procédé de l'invention, on détermine un signal émis par chaque site de la gamme étalon reflétant la quantité de fixation de molécule cible étalon fixée sur chaque site de la gamme étalon. Dans l'étape (ε) du procédé de l'invention, on détermine notamment un signal émis par chaque site d'analyse reflétant la quantité d'analyte fixé sur chaque site d'analyse.

Ces déterminations peuvent être réalisées de différentes manières suivant l'utilisation de marqueurs ou non dans le procédé de l'invention. L'essentiel est de pouvoir attribuer un signal certain et représentatif de la quantité de cibles fixées sur chacun des sites de la gamme étalon et un signal certain et représentatif de la quantité d'analyte fixé sur chaque site d'analyse.

Lorsque des marqueurs sont utilisés, les moyens techniques appropriés pour la détection de ces marqueurs sont utilisés. Si les premier et deuxième moyens de détection utilisés utilisent les mêmes marqueurs, le moyen de détection sera le même pour les différents sites d'analyse et pour les différents sites de la gamme étalon. Ces techniques de lecture de signal sont connues de l'homme du métier. Dans les exemples de marqueurs précités, elles permettent de détecter par exemple une quantité de fluorescence, une quantité de radioactivité, une quantité de produit de la réaction enzymatique, une luminescence, etc. Les documents précités relatifs aux marqueurs présentent des techniques de détermination de ces signaux qui sont utilisables pour la mise en oeuvre du procédé de l'invention.

Selon l'invention, la détermination du signal de chaque site de la gamme étalon et/ou de chaque site d'analyse peut également être réalisée, en particulier lorsque aucun marqueur n'est utilisé, par une méthode choisi dans le groupe comprenant les techniques de détection par résonance plasmonique de surface, les techniques de détection photothermiques, les techniques ellipsométriques, les techniques de détection photométriques et les techniques de détection acoustiques. Ces techniques sont connues de l'homme du métier. Les documents [8] et [9] de la liste de références exposent des techniques de détection photothermiques utilisables pour la mise en oeuvre du procédé de la présente invention pour détecter un signal sans marqueur.

Selon l'invention, il est possible d'utiliser des techniques différentes de détermination des signaux, avec ou sans marqueurs, pour le site d'analyse et pour la gamme étalon. Ainsi, le procédé de l'invention permet de comparer des résultats obtenus avec une méthode sans marquage (site d'analyse ou gamme étalon) avec d'autres résultats obtenus sur la même puce avec marquage (site d'analyse ou gamme étalon), par exemple par fluorescence. L'essentiel est que les conditions opératoires de fixation de l'analyte sur son site d'analyse et de la molécule cible étalon sur la gamme étalon soient effectués simultanément sur la même puce et dans les mêmes conditions opératoires.

Dans l'étape (ε) on rapproche le signal émis par chaque site d'analyse aux différents signaux émis par la gamme étalon pour exprimer ledit signal du site d'analyse en fonction de la proportion P définie ci-dessus. En fait, on compare l'intensité du signal émis par chaque site d'analyse aux différents signaux de la gamme étalon pour en déduire, par rapport à un signal équivalent de la gamme étalon, une valeur de signal rapportée à la proportion P. D'autres rapprochements qui tiennent compte d'autres critères de la gamme étalon sont également possibles (nombre MN, nombre MSE, rapport MN/MSE, rapport MSE/MN, etc.)

Avantageusement, si la nature de l'analyte et de la sonde étalon est la même, il est possible de déterminer directement et de manière fiable et reproductible, par simple lecture, la proportion d'analyte(s) sur chaque site d'analyse. En effet, cette détermination est effectuée grâce à la présente invention au moyen d'une gamme étalon réalisée simultanément, à partir du même échantillon, et donc dans les mêmes conditions opératoires, que pour l'analyte.

Si la nature de l'analyte est différente de celle de la molécule sonde étalon, il est possible de déterminer une valeur relative, mais qui présente toutefois l'avantage d'être stable, reproductible, et d'intégrer les conditions opératoires de l'analyse à celles qui on permis de former simultanément la gamme étalon, sur la même puce, à partir du même échantillon. Cette valeur relative est donc plus fiable que celles qui sont obtenues par les procédés de l'art antérieur.

Dans un exemple particulier de réalisation de la présente invention, lorsque l'analyte, les molécules sondes spécifiques de l'analyte, les molécules sondes étalons, cibles étalons et neutres sont des oligonucléotides, l'étape (γ) de mise en contact a pour objectif l'hybridation sur la puce de l'analyte avec la molécule sonde étalon, et l'hybridation de la molécule cible étalon avec la molécule sonde étalon. Pour cette hybridation, on ajoute à l'échantillon contenant l'analyte un oligonucléotide cible étalon contenant la séquence complémentaire de l'oligonucléotide sonde étalon de la gamme étalon, et marqué préférentiellement, mais non obligatoirement, de la même façon que l'analyte. Cet oligonucléotide est mis en léger excès, de façon à ce que toutes les molécules sondes étalons du site de la gamme étalon comprenant 100% d'oligonucléotide étalon soient hybridées dans les conditions expérimentales d'hybridation utilisées. Ainsi, pour chaque site de la gamme étalon, tous les sites hybridables seront hybridés. On pourra alors exprimer le signal de chaque site d'analyse de la puce à ADN en « % de dilution étalon ». En utilisant dans différentes analyses les mêmes oligonucléotides étalons et non spécifiques (c'est à dire ne reconnaissant et ne fixant pas l'analyte), cette unité est indépendant de l'échantillon, de la chimie de marquage, du marqueur utilisé (vert, rouge ou bleu, fluorescent, photonique, etc.), des conditions d'hybridation (à condition que l'hybridation de l'oligonucléotide étalon soit complète), du lot de puce, de l'efficacité de greffage, etc., ce qui permet de comparer directement les expériences entre elles, sans expérimentations complémentaires comme cela était nécessaire dans les techniques de l'art antérieur.

De manière générale, il est possible de précéder à une lecture directe, ou de faire, à partir de la gamme étalon, une courbe étalon, par exemple (signal déterminé) = f(P), exploitable pour l'analyse des sites d'analyse de ladite puce.

La puce et le procédé de la présente invention sont donc fiables, précis, et les résultats sont reproductibles. La présente invention s'appliquent aux différentes puces à ADN connues et futures ; elle permet de comparer les signaux entre plusieurs expériences, évite d'utiliser une puce à ADN pour chaque point de gamme étalon, et permet d'éviter de répéter constamment des expériences d'étalonnage comme cela est nécessaire avec les procédés de l'art antérieur.

Avantageusement, dans le cas des méthodes de détection avec marquage enzymatique, par exemple la luminescence, la présente invention permet d'éviter à avoir à calibrer l'activité de chaque lot d'enzyme, chez le fabricant comme chez le client. Ceci représente donc une simplification considérable de la « chaîne métrologique ».

La présente invention diminue donc la complexité et les coûts des analyses sur puce, par exemple sur puce biologique, pour les laboratoires, dans l'industrie et dans la recherche.

Etant donné que la présente invention peut s'appuyer sur toutes les puces d'analyse de l'art antérieur mettant en jeu des sites de reconnaissance et de fixation d'un analyte, l'homme du métier comprendra aisément qu'elle trouve une application dans tous les domaines où ce type de puce peut être utilisé.

Ainsi, la présente invention se rapporte également à une trousse de diagnostic comprenant une puce selon l'invention. Dans ce cas, les sites d'analyse de la puce de la présente invention comprennent des molécules sondes spécifiques de reconnaissance de l'analyte recherché pour pouvoir obtenir des résultats qui seront ensuite interprétables pour établir un diagnostic. Par exemple, il peut s'agir de molécules sondes choisies parmi les oligonucléotides, les ADNc, les anticorps, les lectines, les aptamers, etc.

La présente invention se rapporte également à une trousse d'analyse comprenant une puce selon l'invention. Dans ce cas, les sites d'analyse de la puce de la présente invention comprennent des molécules sondes spécifiques de reconnaissance de l'analyte recherché pour pouvoir effectuer ladite analyse. Il peut s'agir d'une trousse d'analyse qualitative ou quantitative. Par exemple, il peut s'agir de molécules sondes choisies parmi les oligonucléotides, les ADNc, les anticorps, les lectines, les aptamers, etc.

La présente invention se rapporte également à l'utilisation d'une puce selon la présente invention pour suivre des variations d'expression de gènes dans des cellules des tissus *in vitro.* Par exemple le document [1] décrit un protocole utilisable sur la puce de la présente invention pour suivre ces variations d'expression de gènes.

La présente invention se rapporte également à l'utilisation d'une puce selon l'invention dans un procédé de génotypage *in vitro*. Par exemple le document [1] décrit un protocole utilisable sur la puce de la présente invention pour la mise en oeuvre d'un procédé de génotypage.

Par exemple, sur l'utilité et pour l'utilisation des puces d'expression de gène selon la présente invention, on peut se reporter à la description accessible dans le document [10] qui décrit des protocoles utilisables pour la mise en oeuvre du procédé de l'invention dans ces applications.

Par exemple, sur l'utilisation de la présente invention dans un procédé de génotypage, par exemple à des fins diagnostiques, on pourra se référer aux documents [11] et [12] qui décrivent chacun des protocoles utilisables pour la mise en oeuvre du procédé de l'invention dans cette application.

D'autres caractéristiques et avantages apparaîtront encore à la lecture des exemples qui suivent donné à titre illustratif en référence aux figures annexées.

## BRÈVE DESCRIPTION DES FIGURES

- La figure 1 est une représentation schématique d'une gamme étalon sur une puce conforme à la présente invention.
- La figure 2 est une représentation schématique d'une gamme étalon sur puce conforme à la présente invention lorsque les molécules cibles étalons sont fixées sur les molécules sondes étalons. Des marqueurs sont également représentés.
- La figure 3 représente deux négatifs d'images d'une gamme étalon sur une puce selon l'invention avec exposition 560 ms, gain 0 (gauche) ou 16 (droite).
- La figure 4 est un graphique présentant des courbes de signaux en fonction de la proportion d'oligonucléotide étalon sur les sites de capture, pour quatre expériences indépendantes (P1, P5, P6 et P7).

Ces courbes constituent des courbes étalon obtenues à partir des gammes étalon conformes à la présente invention.

- La figure 5 est un graphique regroupant les résultats de signaux déterminés pour chacun des sites d'une gamme étalon de la présente invention avec un marquage par des particules fluorescentes.
- La figure 6 est une représentation schématique du montage utilisé par les présents inventeurs pour la fabrication de puces selon la présente invention.
- La figure 7 est un schéma du mécanisme biochimique de marquage indirecte, avec des particules de latex, de la fixation de la molécule cible étalon sur la molécule onde étalon sur une puce de la présente invention.
- La figure 8 est une photographie d'un support de puce utilisé pour fabriquer une puce selon la présente invention.
- La figure 9 est une représentation schématique d'une puce d'analyse selon l'invention permettant de détecter la présence des virus RSV A et RSV B responsables de bronchiolites (RSV pour « Respiratory Syncytial Virus »), et des virus Inf A (influenza A) et Inf B (Influenza B) responsables de la grippe chez un patient.

## EXEMPLES

### Exemple 1 : Puce conforme à la présente invention

#### 1.1 Support de la puce

Le support utilisé pour fabriquer une puce conforme à l'invention est un imageur noir et blanc du type « VV5501 VGA Monochrome Image Sensor » (marque de commerce de ST Microelectronics) dont les principales caractéristiques techniques sont données dans le tableau suivant :

| Format d'image | 640 x 480 pixels (VGA) |
|---|---|
| Taille du pixel | 5,6 $\mu$m x 5,6 $\mu$m |

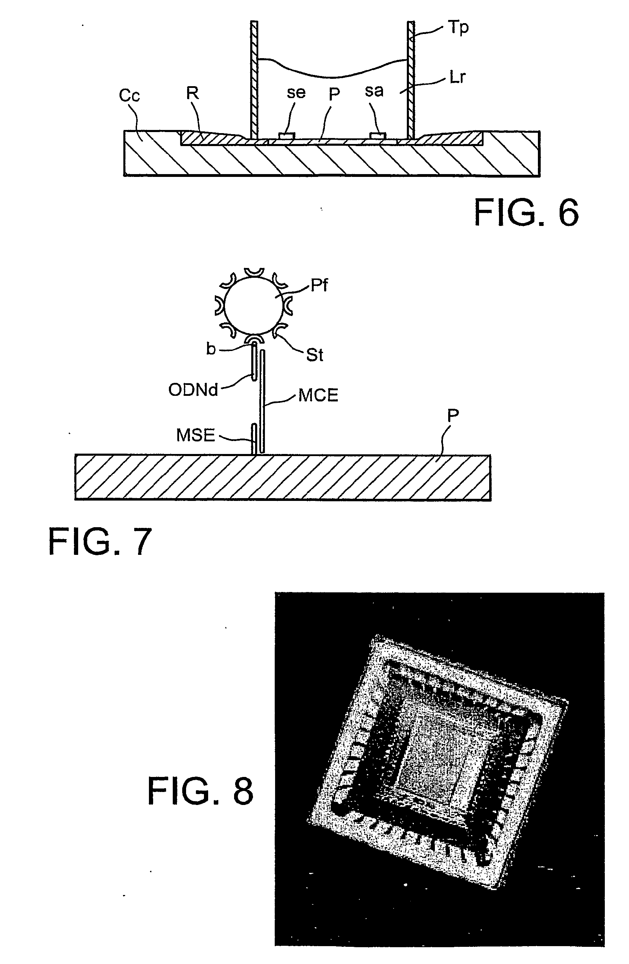

Ce support est représenté en photographie sur en vue du dessus sur la figure 8 annexée. Il comprend en son centre une surface de silicium. La figure 6 décrite ci-dessous est une coupe transversale schématique de cette puce comportant en plus un tube en plastique collé dessus (voir ci-dessous).
Ce support est en fait destiné à la réalisation d'une caméra numérique faible coût comporte des fonctions de contrôle de gain et d'exposition et présente théoriquement un rapport signal sur bruit de 56 dB. De plus, il ne nécessite que très peu de composants externes pour assurer son fonctionnement et dispose d'un kit (logiciel et matériel) permettant la gestion des paramètres internes de la caméra.

#### 1.2 Premières étapes de fabrication de la puce

Des supports de puces comme décrits dans le paragraphe 1.1 précédent ont été modifiés pour pouvoir fonctionnaliser la surface de silicium (voir figure 1). Pour accéder à la surface silicium du photo-détecteur (au centre du support), le capot verre a été ôté et les connexions de liaison (« bonding ») ont été noyées sous une résine de globtop (terme générique désignant toute résine utilisée pour protéger les connections de liaison). La couche de passivation (nitrure de silicium) au centre du photodétecteur a été fonctionnalisée (silanisation + greffage des sondes de capture en sites).
Pour les expériences suivantes de fonctionnalisation de la surface de silicium, un tube en plastique a été collé (colle UV) sur la puce pour définir une chambre réactionnelle au dessus de la surface active, dans laquelle les hybridations, lavages, et révélation ont été réalisés. La figure 6 annexée est une représentation schématique du montage utilisé : il comprend une résine (R) de protection des connections de la puce, la surface de silicium (P), un cadre céramique (Cc), le tube en plastique collé (Tp), et les sites (se = un site étalon, et sa = un site d'analyse) sur lesquels sont fixées soit les molécules sondes et/ou neutres étalons, soit les molécules sondes spécifiques de l'analyte. Sur ce schéma, on montre que le tube est prévu pour contenir un liquide réactionnel (Lr) en contact avec la surface de silicium.
(S) et (Cc) sont visibles en vue du dessus sur la photographie de la figure 8, représentée avant le collage du tube et le dépôt de la résine.

#### 1.3 Fonctionnalisation de la surface de silicium par des molécules sondes et neutres qui sont des oligonucléotides

**1.3.1** Faire apparaître des silanols

(i) On fait d'abord apparaître des silanols à la surface de nitrure de silicium de la puce : Agitation 2h à température ambiante dans la solution : NaOH 1g / eau 3 ml / éthanol 99% 4 ml. Lavages abondants à l'eau. Agitation 1h en HCl 0.2 N, puis rinçages abondants à l'eau. Séchage à 80°C en étuve.
ii) silanisation : les lames sont baignées à température ambiante pendant 24 heures dans du 3-aminopropyltriéthoxysilane à 10% (vol/vol) dans l'éthanol, sous argon. Puis séchage. Puis lavage éthanol 199%, puis un deuxième lavage éthanol 99% + ultrasons. Chaque lavage dure quelques minutes. Séchage. Recuit 3 heures à 110°C à sec.
iii) pre-activation : les lames sont immergées dans une solution de KOH (1,5 g de KOH dans 20 ml d'eau). Incubation 5 à 10 minutes, à température ambiante, avec agitation. Puis rinçage à l'eau pure.
iv) activation : les lames sont immergées dans une solution de glutaraldéhyde (4 ml de glutaraldéhyde mis dans 16 ml d'eau). Incubation 1h30 à température ambiante. Rinçage à l'eau pure. Séchage.

**1.3.2** Immobilisation des oligonucléotides

Après activation de ces groupements $NH_2$ par du glutaraldéhyde, des oligonucléotides (ODN dans la suite du texte) aminés peuvent être déposés sur les différents sites de la surface de la puce pour leur greffage, au moyen d'un robot de la société Karl Züss équipé d'une pipette de la société Mikrodrop. Le diamètre des sites a été évalué à environ 140 $\mu$m.

Préparation des solutions d'oligonucléotides à déposer : les solutions utilisées comprennent chacune 10 $\mu$M d'ODN au total (sonde + neutre) à déposer sur chaque site de la gamme étalon de la puce. Elles contiennent un mélange d'ODN sonde étalon et d'ODN neutre (non spécifique) en proportions 100% étalon, 10% étalon, 1% étalon, 0.1% étalon, et 100% non spécifique, soit une gamme étalon fabriquée avec 5 sites ou points. Ces 5 solutions ont été déposées sur 5 lignes de sites au robot de la société Karl Züss, muni d'une pipette piezo électrique de la société Mikrodrop. Les sites sont déposés au pas de 200 $\mu$m. L'écart inter-ligne est de 400 $\mu$m environ. Le diamètre des sites est d'environ 140 $\mu$m.

Tous les oligonucléotides de cette expérience sont longs de 22 bases. Les séquences utilisées sont les suivantes :

- Séquence de l'oligonucléotide sonde (SEQ ID n°1) :

  5' ($H_2$N-)ATGAACAAGTAGATAAATTAGT 3'

- Séquence de l'oligonucléotide neutre (SEQ ID n°2) :

  5' ($H_2$N-)CTAAAGGAATAGTGTAAATAAT 3'

Les groupements -$NH_2$ servent au greffage sur les aldéhydes du glutaraldéhyde utilisé dans cet exemple.

(la séquence de l'oligonucléotide cible étalon est décrite dans l'exemple 3)

**1.4 Hybridation et marquage**

Ces puces ont été hybridées avec une solution à 15 nM de conjugué HRP à 37°C pendant 30 minutes, lavées, puis imagées en présence de mélange substrat Pierce (« Super ELISA Femto Maximum Sensitivity Kit »)(marque de commerce). Le conjugué HRP est un oligonucléotide complémentaire de l'oligonucléotide sonde, auquel l'enzyme HRP a été couplé (« Horse Radish Peroxydase »).

Toutes les hybridations ont été réalisées, en chambre humide, et à 37 °C pendant 30 minutes dans TE 1X, NaCl 1 M, Triton X-100 0.05%. Le volume d'hybridation est de 200 $\mu$l (soit une veine liquide d'environ 3 mm d'épaisseur). Il n'y a pas d'agitation. L'hybridation est suivie de 3 lavages de 400 $\mu$l en TE 1X, NaCl 1 M, Triton X-100 0.05%, puis un lavage en TE 1X, NaCl 1 M. TE 1X signifie : Tris 10 mM, EDTA 1 mM, pH 8.

Après hybridation et lavages, la puce est montée sur une carte de lecture.

**1.5 Détermination des signaux de la gamme étalon et lecture**

Le milieu de rinçage est ôté, puis l'on ajoute 200 $\mu$l du mélange substrat SuperSignal ELISA Femto Maximum Sensitivity Substrate (Pierce, #37075 (marque de commerce)).

Acquisition d'images: 50 images en gain 15 diviseur 15 (1.8 images/s), 50 images gain 0 diviseur 15 (1.8 images/s). La première série d'image permet de visualiser les signaux faibles. La seconde permet de visualiser les signaux forts, qui saturent le capteur dans les conditions précédentes. Les 50 images de luminescence sont moyennées pixel à pixel, ainsi que les 50 images de noir. Puis la moyenne des images de noir est soustraite à la moyenne des images de luminescence. Sur l'image résultante obtenue, on prend la moyenne de l'intensité de chaque site, auquel on soustrait la valeur moyenne du bruit de fond autour du site. La valeur obtenue représente le signal de luminescence du site.

En effet, la dynamique du capteur (estimée théoriquement à 320 ne suffit pas à couvrir la dynamique de signal généré par la biologie. Il a donc fallu réaliser deux acquisitions avec des réglages de gain ou d'exposition différents. Les conditions choisies (gain 0/exposition 560 ms, et gain 16/exposition 560 ms) permettent d'obtenir une image non saturée pour les signaux forts et une image des signaux faibles : photographies de la figure 3, à gauche gain 0 et à droite gain 16. Sur l'image de droite, on peut nettement distinguer, avant même traitement de l'image, quatre lignes horizontales de sites (« spots »), de haut en bas : 100%, 10%, 1%, 0,1% étalon suivies d'une ligne de sites ne comprenant pas de molécule sonde étalon, mais uniquement la molécule neutre (non spécifique) qui n'est pas visible.

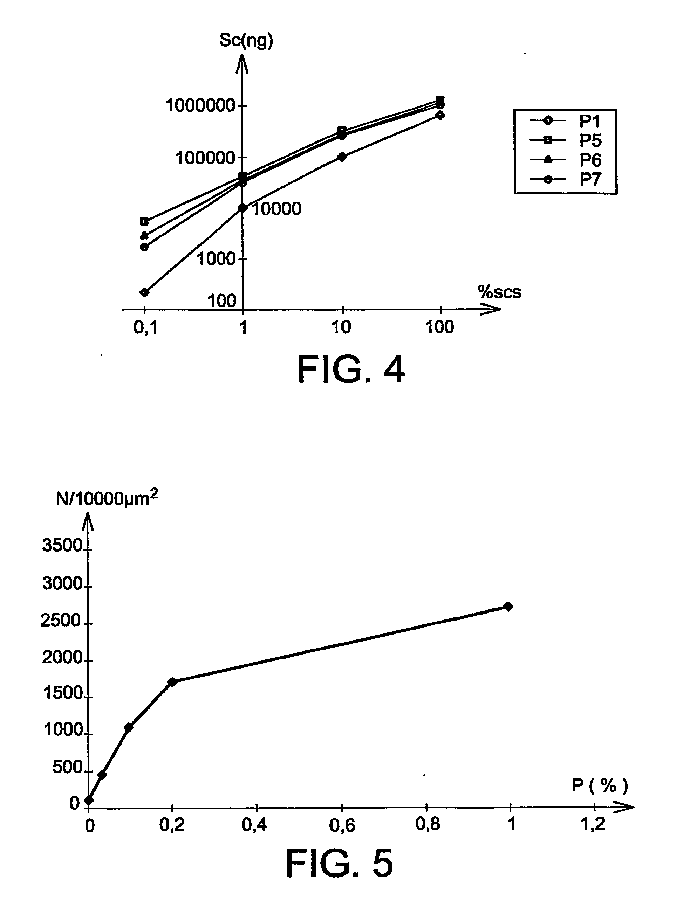

Les niveaux de gris (ng) des sites sur les images traitées ont été quantifiés, puis normalisés et les valeurs obtenues ont été reportées sur le graphique représenté sur la figure 4 annexée. Sur cette figure P1 P5, P6 et P7 (P pour puce) représentent quatre expériences indépendantes sur quatre puces différentes et conformes à la présente invention. En ordonnée, on représente le signal corrigé (Sc) (en ng), et en abscisse, on représente le pourcentage de molécule sonde capturée (% SCS).

On peut observer sur ce graphique que la détection par hybridation de cibles étalons couplées à une enzyme permet de distinguer des concentrations surfaciques de chaque site de la gamme étalon (ici sondes étalons de capture spécifiques de la cible étalon) sur une gamme utile de 1000 (de 0,1% à 100%).

Ce système mime une variation de la concentration surfacique de sites de cibles étalons capturées. On peut donc détecter les cibles avec une gamme de concentrations, c'est à dire avoir une dynamique de détection, de l'ordre de 1000.

Dans cet exemple, réalisé pour la démonstration, la puce est hybridée avec un oligonucléotide directement couplé à l'enzyme HRP. Dans les applications réelles, il y aura bien sûr sur la puce d'autres sites que les seuls sites étalon. La puce sera donc par exemple hybridée avec l'échantillon préalablement marqué par un haptène, par exemple la biotine. A l'échantillon, pendant l'hybridation, on ajoutera un oligonucléotide complémentaire de l'oligonucléotide étalon, et marqué à la biotine. Après l'hybridation, on incubera un conjugué streptavidineHRP qui se fixera aux biotines présentes sur la puce. Puis, on procédera à la révélation par luminescence comme décrit ci-dessus. C'est cette méthode « en deux temps » qui a été utilisée dans l'exemple suivant, mais avec des particules comme marqueurs, et non des enzymes.

**Exemple 2 : marquage direct de la molécule cible étalon par particules fluorescentes**

Dans cet exemple, la molécule cible étalon (MCE) hybridée sur la puce (P) est marquée avec un haptène, la biotine (b), en l'occurrence au moyen d'un ODN de détection (ODNd). Puis une étape de "coloration" a lieu, en incubant la surface de la puce avec des particules fluorescentes (Pf) fonctionnalisées à la streptavidine (St), qui vont donc se fixer aux biotines (b). Les séquences sont indiquées ci-dessous. La figure 7 représente schématiquement le mécanisme biochimique utilisé dans ce procédé, sur la surface (S) de la puce : un ODN de capture est fixé sur le support formant la puce et joue le rôle de l'oligonucléotide sonde étalon (MSE) de l'invention ; l'ensemble cible + ODN de détection joue le rôle de l'oligonucléotide complémentaire (cible) (MCE). L'ODN de capture (sonde) est constitué de 70 nucléotides. La cible est constituée de 513 nucléotides. L'ODN de détection est constitué de 16 nucléotides. Les séquences sont les suivantes :

- Séquence de l'oligonucléotide sonde (SEQ IDn°3) :

```
5' TCACTATTAT CTTGTATTAC TACTGCCCCT TCACCTTTCC AGAGGAGC
TT TGCTGGTCCT TTCCAAAGTG 3'
```

- Séquence de l'oligonuléotide neutre (SEQ IDn°4) :

```
5' ACTGTTACTG ACCTACCATT TGTTACCTAT GCTAAGCTCA TTGCACCT
CT GATTGCCGAG GCCTTTCTTT 3'
```

- Séquence de l'oligonuléotide cible (SEQ IDn°5) :

```
5' ACAGCAGTAC AAATGGCAGT ATTCATCCAC AATTTTAAAA GAAAAGGG
GG GATTGGGGGG TACAGTGCAG GGGAAGAAT AGTAGACATA ATAGCAAC
AG ACATACAAAC TAAAGAATTA CAAAAACCCT TACAAAAATT CAAAATTT
TC GGGTTTATTA CAGGGACAGC AGAAATCCAC TTTGGAAAGG ACCAGCAA
AG CTCCTCTGGA AAGGTGAAGG GGCAGTAGTA ATACAAGATA ATAGTGAC
AT AAAAGTAGTG CCAAGAAGAA AAGCAAAGAT CATTAGGGAT TATGGAAA
AC AGATGGCAGG TGATGATTGT GTGGCAAGTA GACAGGATGA GATTAGAA
CA TGGAAAAGTT TAGTAAAACA CCATATGTAT GTTTCAGGGA AAGCTAGG
GG TAGGTTTTAT AGACATCACT ATGAAAGCCC TCATCCAAGA ATAAGTTC
AG AAGTAAATCG AATTCCCGCG GCCATGGCGG CCGGGAGCAT GCGACGTC
GG GCCCAATTCG CCC 3'
```

Séquence de l'oligonuléotide de détection (ODNd) (SEQ IDn°6) :

5' TTCTGAACTTATTCTT 3'

Le rendement de cette étape de coloration est encore mal compris, et varie selon les conditions expérimentales, la chimie de surface utilisée pour la fabrication de la puce à ADN, les particules fluorescentes utilisées, etc. Ainsi, bien que l'on puisse compter les particules individuellement, il faut tout de même, dans les procédés de l'art antérieur, calibrer les mesures pour pouvoir comparer les expériences entre elles.

D'où l'intérêt de la présente invention pour ce type de marquage également, car il permet avantageusement de se passer de ce calibrage.

Le support formant la puce dans cet exemple est une lame de microscope, 25 x 75 mm. Pour toutes les opérations, la lame de microscope est immergée dans un petit bécher contenant la solution désirée. Les opérations sont les suivantes :

On applique à ce support les étapes (i) à (iv) du paragraphe 1.3.1 de l'exemple 1 ci-dessus. Puis :

(1) Immobilisation des oligonucléotides sondes et neutres précités : Dépôts manuels, à la micropipette, d'oligonucléotides aminés à 10 $\mu$M, en goutte de 3 $\mu$l. La taille des sites est d'environ 3 mm. Incubation en chambre humide une nuit à température ambiante.

Préparation des solutions d'oligonucléotides à déposer : le mélange 10 $\mu$M d'ODN de capture déposé contient un mélange d'ODN étalon et non spécifique en proportions 100% étalon, 1% étalon, 0,2% étalon, 0,1% étalon, 0,033% et 100% molécule neutre (non spécifique) (soit une gamme étalon fabriquée avec 6 points). Ces 6 solutions ont été déposées manuellement.

(2) Traitement post-immobilisation. Réduction des aldéhydes encore présentes : eau 30 ml / NaBH4 105 mg ; incubation 1h à température ambiante, sans agitation. Rinçage à l'eau 5 min, puis avec 5% sodium dodécyl sulfate 5 min, puis à nouveau en eau pure 5 min. Séchage.

(3) Hybridation de la puce et détection : les puces ont été hybridées avec la molécule cible précités de 513 bases (SEQ IDn°5) à 100 nM pendant 30 min à température ambiante en TE NaCl 1M Triton 0,05%. Puis ces puces ont été hybridées avec une solution à 200 nM de l'oligonucléotide de détection à température ambiante pendant 1 heure, puis lavées. Cet oligonucléotide est marqué à la biotine. Ensuite, la lame a été incubée avec des particules fluorescentes neutravidine de 100 nm de diamètre (Molecular Probes, T8861).

Hybridation : toutes les hybridations ont été réalisées à température ambiante en TE 1X, NaCl 1 M, Triton X-100 0.05%. Il n'y a pas d'agitation. L'hybridation est suivie de 3 lavages en TE 1X, NaCl 1 M, Triton X-100 0.05%, puis un lavage en TE 1X, NaCl 1 M. TE 1X signifie : Tris 10 mM, EDTA 1 mM, pH 8.

(4) Révélation et lecture : l'incubation des particules fluorescentes est réalisée en TE NaCl 1 M, à une concentration de $10^6$ particules par $\mu$l pendant 3h, à température ambiante. Les particules ont un diamètre de 100 nanomètres.

(5) Extraction des résultats numériques : chaque site est imagé au microscope à fluorescence (cube fluorescéine). Puis on compte la densité de particules sur chaque site, soit manuellement, soit en automatique à l'aide d'une routine matlab (marque de commerce). Les résultats entre ces deux méthodes sont cohérents.

Une gamme étalon utilisable est bien obtenue. Elle est représentée par le graphique de la figure 5 annexée, sur lequel l'ordonnée représente le nombre de particules pour 10000μm², et sur lequel l'abscisse représente P (% MSE/ (MN+MSE)).

En utilisant, à la place du marqueur précité, des particules fluorescentes Quantum Dot (marque de commerce) de la société Quantum Dot Corp., on peut s'attendre à obtenir un rendement de marquage bien meilleur que celui obtenu ici par les latex fluorescents.

**Exemple 3 : analyse d'un échantillon**

Nous proposons comme exemple une puce simple dédiée à un premier criblage de patients présentant une infection des voies respiratoires supérieures. La puce vise plus précisément à détecter la présence des virus RSV A et RSV B, responsables de la bronchiolite, et celle des virus Influenza A (Inf A) et Influenza B (Inf B), responsables de la grippe.

Dans tous les cas, il s'agit de virus à ARN, très polymorphes ; les symptômes sont relativement proches, et ces virus peuvent être dangereux chez les nourrissons, chez les personnes âgées, chez tous les immuno-déprimés d'une façon générale.

La puce que nous utiliserons sera exactement de la même technologie que celle de l'exemple 1, avec une chimie de fonctionnalisation identique à celle de l'exemple 2.

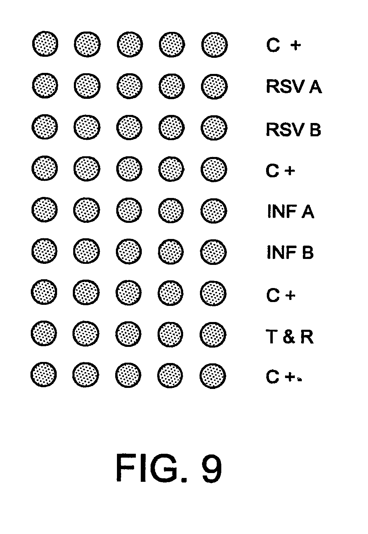

Les sites (d'analyse ainsi que ceux de la gamme étalon) ont un diamètre de 160 μm environ, et sont disposés au pas de 300 μm. La figure 9 annexée représente schématiquement cette puce conforme à la présente invention. Les sites sont représentés circulaires.

Pour RSV A, RSV B, Inf A et Inf B, ainsi que pour le contrôle « stain » (coloration) et révélétion (T & R), c'est le même site qui est répété cinq fois.

Les molécules sur les sites de contrôle de « stain » et révélation (T & R) est un oligonucléotide identique aux molécules MCE, déjà marquées, à la fabrication des oligonucléotides, par une biotine.

Le contrôle positif (C +) est une gamme étalon, au sens de l'invention, dans les proportions 100%, 10%, 1%, 0,1%, 0% (de gauche à droite).

La puce sera utilisée de la façon suivante :

a) le biologiste purifie les ARNs des échantillons selon les méthodes de l'art.

b) on mélange à l'échantillon biologique, la molécule cible étalon marquée à la biotine à la fabrication.

c) on marque l'échantillon avec la biotine, selon l'une des variantes de la méthode LDC ayant fait l'objet de demandes de brevets déposées par BioMérieux dans les familles de brevets correspondantes aux documents [13], [14], [15] ou [16] de la liste de référence.

d) on incube l'ensemble sur la puce (étape d'hybridation), puis lavages, comme pour l'exemple 1.

e) on ajoute le conjugué enzymatique streptavidine/HRP, comme dans l'exemple 1. A cette étape, le conjugué s'accroche aux analytes présents sur les sites d'analyse, ainsi qu'aux molécules MCE présentes sur les sites de la gamme étalon, ainsi qu'aux molécules des sites du contrôle « stain » et révélération.

f) on procède à la révélation par luminescence comme dans l'exemple 1.

Les séquences des oligonucléotides MCE, neutre et MSE sont identiques à celle de l'exemple 1.

**Liste de références**

**[1]** François GELI, « Puces à ADN et autres systèmes d'analyse », Biofutur n°206, Le technoscope de Biofutur, pages 1-14, décembre 2000.

**[2]** Cahill DJ, Nordhoff E., "protein arrays and their role in proteomics", Adv. Biochem. Eng. Biotechnol., 2003, 83, 177-187.

**[3]** Lopez MF, Pluskal MG, "Protein micro- and macroarrays : digitizing the proteome", J. Chromatogr. B Analyt. Technol. Biomed. Life Sci., 2003, 787(1), 19-27.

**[4]** EP-A-1 119 769.

**[5]** FR-A-2 784 189.

**[6]** WO-A-03/023061.

**[7]** Bar-coding biomolecules with fluorescent nanocrystals, Nature Biotechnology, 2001, 19, 621-622.

**[8]** FR-A-2 799 281.

**[9]** FR-A-2 799 282.

**[10]** Bertrand Jordan, "voyage au pays des puces", médecine/sciences, 1998, 14, 1097-1102.

**[11]** Troesch A., et al., "Mycobacterium species identification and rifampin resistance testing with high-density DNA probe array", J. Clin. Microbiol., 1999, 37, 49-55.

**[12]** Christen R. et Mabilat C., "Applications des puces à ADN en bactériologie", Bull. Soc. Fr. Microbiol., 1998, 13, 10-17.

**[13]** « Procédé de marquage d'un acide ribonucléique et fragments d'ARN marqués ainsi obtenus », FR 98 07870 et ses extensions dans les autres pays (fammille de brevets). Inventeur : A. Laayoun.

**[14]** « Process for labelling a nucleic acid », EP-A-1 238 116. Inventeurs : A. Banerjee, A. Laayoun, M. Becker, K. Browne, M. Friedenberg, F. Hajjar.

**[15]** « Process for labelling a nucleic acid and labelled RNA fragments which are obtained thereby », WO-A-01/44506. Inventeurs : A. Laayoun, D. Do, C. Miyada.

**[16]** « Procédé de marquage et de fragmentation d'AND », FR n°01 06039. Inventeurs : J. Lhomme, E. Trevisiol, A. Laayoun, C. Bourget, M. Kotera, C. Tora.

[17]: SCHUCHHARDT J ET AL: "NORMALIZATION STRATEGIES FOR CDNA MICROARRAYS" NUCLEIC ACIDS RESEARCH, OXFORD UNIVERSITY PRESS, SURREY, GB, vol. 28, no. 10, 15 mai 2000 (2000-05-15), pages E47I-E47V.

**Revendications**

1. Puce d'analyse d'au moins un analyte présent dans un échantillon liquide, ladite puce comprenant :

   a) au moins un site d'analyse dudit, au moins un, analyte, ledit site d'analyse étant disposé sur la puce de façon à permettre la reconnaissance et la fixation spécifique de l'analyte ; et
   b) une gamme étalon (G), ladite gamme étalon étant constituée de plusieurs sites étalons disposés chacun sur ladite puce de manière déterminée et indépendants les uns des autres, chaque site étalon de cette gamme comportant, fixé(s) à sa surface et en proportion déterminée P différente pour chaque site par rapport aux autres sites étalons de ladite gamme : (i) au moins une molécule sonde étalon (MSE) permettant de reconnaître et de se fixer spécifiquement à une molécule cible étalon (MCE) déterminée , et (ii) au moins une molécule neutre (MN) incapable de reconnaître et de se fixer à ladite molécule cible étalon, la molécule sonde étalon et la molécule neutre étant toutes les deux incapables de reconnaître et de fixer ledit ou lesdits analyte(s) ; avec

   $$P = \frac{\text{nombre de MSE}}{\text{nombre de MSE} + \text{nombre de MN}}$$ et $0 \leq P \leq 1$, la somme de nombre d'MSE + nombre d'MN étant constante d'un site étalon à l'autre.

2. Puce selon la revendication 1, dans laquelle l'analyte et la molécule cible étalon sont de même nature.

3. Puce selon la revendication 2, dans laquelle, l'analyte et la molécule cible étalon sont des oligonucléotides ou des anticorps.

4. Puce selon la revendication 1, dans laquelle la molécule sonde étalon, la molécule cible étalon et la molécule neutre sont des oligonucléotides.

**5.** Puce selon la revendication 1 ou 4, dans laquelle le, au moins un, analyte est un acide nucléique, et dans laquelle le, au moins un, site d'analyse est un site fonctionnalisé par un acide nucléique complémentaire à celui-ci.

**6.** Puce selon la revendication 1, dans laquelle les sites de la gamme étalon sont disposés sur la puce de manière linéaire et sont ordonnés en fonction de la proportion P de chaque site.

**7.** Trousse de diagnostic comprenant une puce selon la revendication 1.

**8.** Trousse d'analyse comprenant une puce selon la revendication 1.

**9.** Utilisation d'une puce selon la revendication 1 pour suivre des variations d'expression de gènes dans des cellules ou des tissus *in vitro.*

**10.** Utilisation d'une puce selon la revendication 1 dans un procédé de génotypage *in vitro.*

**11.** Procédé d'analyse *in vitro* d'au moins un analyte susceptible d'être présent dans un échantillon liquide comprenant les étapes suivantes :

($\alpha$) optionnellement, fixation sur l'analyte d'un premier moyen de détection ;
($\beta$) addition dans l'échantillon à analyser, comprenant l'analyte optionnellement marqué, d'une molécule cible étalon (MCE) sur laquelle un deuxième moyen de détection, identique ou différent du premier moyen de détection, a optionnellement été fixé, ladite MCE permettant de reconnaître et de se fixer spécifiquement à la molécule sonde étalon (MSE) d'une puce d'analyse selon la revendication 1, ladite MCE étant ajoutée audit échantillon en une quantité suffisante pour saturer les MSE de la gamme étalon (G) de ladite puce, créant ainsi une gamme étalon fonction de la proportion déterminée P différente pour chaque site étalon ;
($\gamma$) mise en contact de l'échantillon à analyser comprenant l'analyte et la MCE optionnellement marqués avec ladite puce d'analyse selon la revendication 1 comprenant au moins un site d'analyse dudit au moins un analyte dans des conditions physicochimiques telles que : d'une part l'analyte à analyser, s'il est présent, soit fixé sur son site d'analyse sur la puce ; et d'autre part la MCE reconnaisse spécifiquement la MSE et se fixe à celle-ci sur les différents sites étalon de la gamme étalon de la puce ;
($\delta$) détermination d'un signal étalon émis, le cas échéant grâce au deuxième moyen de détection, par chaque site étalon de la gamme étalon, ledit signal étant fonction de la quantité de molécule cible étalon fixée sur celui-ci ; et
($\epsilon$) détermination d'un signal d'analyse émis, le cas échéant grâce au premier moyen de détection, par ledit, au moins un, site d'analyse et fonction de la quantité d'analyte fixé par le site d'analyse, et rapprochement aux signaux déterminés à l'étape ($\delta$) pour exprimer ce signal d'analyse en fonction de P.

**12.** Procédé selon la revendication 11, dans lequel l'analyte et la molécule cible étalon sont de même nature.

**13.** Procédé selon la revendication 12, dans lequel l'analyte et la molécule cible étalon sont des oligonucléotides ou des anticorps.

**14.** Procédé selon la revendication 11, dans lequel la molécule sonde étalon, la molécule cible étalon et la molécule neutre sont des oligonucléotides.

**15.** Procédé selon la revendication 11 ou 14, dans lequel le, au moins un, analyte est un acide nucléique, et dans lequel le, au moins un, site d'analyse est un site fonctionnalisé par un acide nucléique complémentaire à cet acide nucléique.

**16.** Procédé selon la revendication 11 ou 15, dans lequel le premier et le deuxième moyen de détection impliquent respectivement un premier et un deuxième marqueurs.

**17.** Procédé selon la revendication 16, dans lequel le premier et le deuxième marqueurs sont choisis indépendamment dans le groupe constitué des marqueurs fluorescents, des marqueurs radioactifs, des marqueurs enzymatiques, des particules de latex, des cristaux photoniques et de l'or colloïdal.

**18.** Procédé selon la revendication 11, dans lequel la détermination du signal de chaque site de la gamme étalon et/ou de chaque site d'analyse est réalisée par une méthode choisi dans le groupe comprenant les techniques de détection par résonance plasmonique de surface, les techniques de détection photothermiques, les techniques ellipsométriques et les techniques de détection photométriques.

**19.** Procédé selon la revendication 11, dans lequel l'analyse est une détection ou un dosage.

**Claims**

**1.** Analysis chip of at least one analyte present in a liquid sample, said chip comprising:

a) at least one analysis spot of said, at least one, analyte, said analysis spot being arranged on the chip in such a way as to permit the recognition and immobilization specific to the analyte; and

b) a reference range (G), said reference range comprising several reference spots each arranged on said chip in a defined manner and independently of one another, each reference spot of this range comprising, immobilized on its surface and in a defined proportion P different for each spot relative to the other reference spots of said range: (i) at least one probe reference molecule (PRM) for recognizing and specifically immobilizing a defined target reference molecule (TRM), and (ii) at least one inert molecule (IM) incapable of recognizing and attaching to said target reference molecule, the probe reference molecule and the inert molecule both being unable to recognize and immobilize said analyte or said analytes; with $P = \dfrac{\text{number of PRMs}}{\text{number of PRMs} + \text{number of IMs}}$

and $0 \leq P \leq 1$, the sum of the number of PRMs + number of IMs being constant from one reference spot to another.

**2.** Chip according to Claim 1, in which the analyte and the target reference molecule are of the same nature.

**3.** Chip according to Claim 2, in which the analyte and the target reference molecule are oligonucleotides or antibodies.

**4.** Chip according to Claim 1, in which the probe reference molecule, the target reference molecule and the inert molecule are oligonucleotides.

**5.** Chip according to Claim 1 or 4, in which the, at least one, analyte is a nucleic acid, and in which the, at least one, analysis spot is a spot functionalized by a nucleic acid complementary to the latter.

**6.** Chip according to Claim 1, in which the spots of the reference range are disposed on the chip linearly and are arranged as a function of the proportion P of each spot.

**7.** Diagnostic kit comprising a chip according to Claim 1.

**8.** Analysis kit comprising a chip according to Claim 1.

**9.** Use of a chip according to Claim 1 for monitoring variations in gene expression in cells or tissues *in vitro.*

**10.** Use of a chip according to Claim 1 in a method of genotyping *in vitro.*

**11.** Method of *in vitro* analysis of at least one analyte that may be present in a liquid sample comprising the following stages:

(α) optionally, immobilization of a first detecting means on the analyte;

(β) addition, to the sample to be analysed, comprising the optionally labelled analyte, of a target reference molecule (TRM) on which a second detecting means, identical to or different from the first detecting means, has optionally been immobilized, said TRM being capable of recognizing and of binding specifically to the probe reference molecule (PRM) of an analysis chip according Claim 1, said TRM being added to said sample in a sufficient amount to saturate the PRM of the reference range (G) of said chip, thus creating a reference range that is a function of the defined proportion P which is different for each reference spot;

(γ) bringing the sample to be analysed comprising the analyte and the TRM, optionally labelled, into contact with said analysis chip according to Claim 1 comprising at least one analysis spot of said at least one analyte in physicochemical conditions such that: on the one hand, the analyte to be analysed, if it is present, binds to its analysis spot on the chip; and on the other hand, the TRM specifically recognizes the PRM and binds to the latter on the various reference spots of the reference range of the chip;

(δ) determination of a reference signal emitted, if applicable owing to the second detecting means, by each reference spot of the reference range, said signal being a function of the amount of target reference molecule immobilized on the latter; and

(ε) determination of an analysis signal emitted, if applicable owing to the first detecting means, by said, at least one, analysis spot and being a function of the amount of analyte immobilized by the analysis spot, and comparison with the signals determined in stage (δ) for expressing this analysis signal as a function of P.

12. Method according to Claim 11, in which the analyte and the target reference molecule are of the same nature.

13. Method according to Claim 12, in which the analyte and the target reference molecule are oligonucleotides or antibodies.

14. Method according to Claim 11, in which the probe reference molecule, the target reference molecule and the inert molecule are oligonucleotides.

15. Method according to Claim 11 or 14, in which the, at least one, analyte is a nucleic acid, and in which the, at least one, analysis spot is a spot functionalized by a nucleic acid complementary to this nucleic acid.

16. Method according to Claim 11 or 15, in which the first and the second detecting means involve respectively a first marker and a second marker.

17. Method according to Claim 16, in which the first and the second markers are selected independently from the group comprising fluorescent markers, radioactive markers, enzymatic markers, latex particles, photonic crystals and colloidal gold.

18. Method according to Claim 11, in which the determination of the signal of each spot of the reference range and/or of each analysis spot is performed by a method selected from the group comprising methods of detection by surface plasmon resonance, methods of photothermal detection, ellipsometric techniques and methods of photometric detection.

19. Method according to Claim 11, in which the analysis is a detection or a quantitative analysis.

**Patentansprüche**

1. Chip zur Analyse wenigstens eines in einer Probe präsenten Analyten, umfassend:

a) wenigstens einen Analyseplatz des genannten wenigstens einen Analyten, wobei der genannte Analyseplatz auf dem Chip so angeordnet ist, dass das Erkennen und das spezifische Fixieren des Analyten möglich ist; und
b) eine Referenzskala (G), wobei diese Referenzskala durch mehrere Referenzplätze gebildet wird, jeder auf bestimmte Weise und unabhängig von den anderen auf dem genannten Chip angeordnet, wobei jeder Analyseplatz dieser Skala, fixiert auf ihrer Oberfläche und mit bestimmter, für jeden Platz in Bezug auf die anderen Skalenplätze der genannten Skala unterschiedlicher Proportion P, umfasst: (i) wenigstens ein Referenz-Sondenmolekül (MSE), fähig ein bestimmtes Referenz-Zielmolekül (MCE) zu erkennen und sich spezifisch an ihm zu fixieren, und (ii) wenigstens ein neutrales Molekül (MN), unfähig das genannte Referenz-Zielmolekül zu erkennen und sich an ihm zu fixieren, wobei das Referenz-Sondenmolekül und das neutrale Molekül alle beide unfähig sind, den oder die genannten Analyten zu erkennen und zu fixieren; mit

$$P = \frac{\text{Anzahl MSE}}{\text{Anzahl MSE} + \text{Anzahl MN}}$$ und $0 \leq P \leq 1$, wobei die Summe von Anzahl MSE + Anzahl MN von einem Referenzplatz zum anderen konstant ist.

2. Chip nach Anspruch 1, bei dem der Analyt und das Referenz-Zielmolekül von derselben Art sind.

3. Chip nach Anspruch 2, bei dem der Analyt und das Referenz-Zielmolekül Oligonukleotide oder Antikörper sind.

4. Chip nach Anspruch 1, bei dem das Referenz-Sondenmolekül, das Referenz-Zielmolekül und das neutrale Molekül Oligonukleotide sind.

5. Chip nach Anspruch 1 oder 4, bei dem der wenigstens eine Analyt eine Nukleinsäure und der wenigstens eine Analyseplatz ein durch eine zu dieser komplementäre Nukleinsäure funktionalisierter Platz ist.

6. Chip nach Anspruch 1, bei dem die Plätze der Referenzskala auf dem Chip in linearer Weise und in Abhängigkeit von der Proportion P jedes Platzes angeordnet sind.

7. Diagnose-Kit mit einem Chip nach Anspruch 1.

8. Analyse-Kit mit einem Chip nach Anspruch 1.

9. Verwendung eines Chips nach Anspruch 1, um Ausdrucksveränderungen von Genen in Zellen oder Geweben *in vitro* zu verfolgen.

10. Verwendung eines Chips nach Anspruch 1 in einem In-vitro-Gentypisierungverfahren.

11. Verfahren zur In-vitro-Analyse wenigstens eines in einer flüssigen Probe vermuteten Analyten, folgende Schritte umfassend:

   ($\alpha$) optional, Fixieren eines ersten Detektionsmittels an dem Analyten;
   ($\beta$) Beigeben - der den optional markierten Analyten enthaltenden Probe - eines Referenz-Zielmoleküls (MCE), an dem optional ein zweites Detektionsmittel, identisch oder unterschiedlich zum ersten Detektionsmittel, fixiert worden ist, wobei das genannte MCE ermöglicht, das Referenz-Sondenmolekül (MSE) eines Analyse-Chips nach Anspruch 1 zu erkennen und sich spezifisch an ihm zu fixieren, wobei das genannte MCE der genannten Probe in einer ausreichenden Menge beigegeben wird, um die MSEs der Referenzskala (G) des genannten Chips zu sättigen und so eine Referenz-Skala herzustellen, die abhängig ist von der für jeden Referenzplatz unterschiedlichen bestimmten Proportion P;
   ($\gamma$) Herstellen des Kontakts der den zu analysierenden Analyten und das optional markierte MCE enthaltenden Probe mit dem genannten Analyse-Chip nach Anspruch 1, umfassend wenigstens einen Platz zur Analyse des genannten wenigstens einen Analyten unter physikalisch-chemischen Bedingungen wie folgt: einerseits ist der zu analysierende Analyt, wenn präsent, auf seinem Analyseplatz auf dem Chip fixiert; und andererseits erkennt das MCE spezifisch das MSE und fixiert sich in den verschiedenen Referenzplätzen der Referenzskala des Chips an diesem;
   ($\delta$) Bestimmen eines gegebenenfalls dank des zweiten Detektionsmittels durch jeden Referenzplatz der Referenzskala gesendeten Referenzsignals, wobei das genannte Signal abhängig ist von der Menge der an ihm fixierten Referenz-Zielmoleküle; und
   ($\varepsilon$) Bestimmen eines gegebenenfalls dank des ersten Detektionsmittels durch den genannten wenigstens einen Analyseplatz gesendeten Analysesignals in Abhängigkeit von der durch den Analyseplatz fixierten Analytmenge, und Annäherung an die in Schritt ($\delta$) bestimmten Signale, um dieses Analysesignal in Abhängigkeit von P auszudrücken.

12. Verfahren nach Anspruch 11, in dem der Analyt und das Zielmolekül von derselben Art sind.

13. Verfahren nach Anspruch 12, in dem der Analyt und das Zielmolekül Oligonukleotide oder Antikörper sind.

14. Verfahren nach Anspruch 11, in dem das Referenz-Sondenmolekül, das Referenz-Zielmolekül und das neutrale Molekül Oligonukleotide sind.

15. Verfahren nach Anspruch 11 oder 14, bei dem der wenigstens eine Analyt eine Nukleinsäure und der wenigstens eine Analyseplatz ein durch eine zu dieser komplementäre Nukleinsäure funktionalisierter Platz ist.

16. Verfahren nach Anspruch 11 oder 15, in dem das erste und das zweite Detektionsmittel jeweils einen ersten und einen zweiten Marker implizieren.

**17.** Verfahren nach Anspruch 16, in dem der erste und der zweite Marker unabhängig aus der Gruppe ausgewählt werden, die gebildet wird durch fluoreszierende Marker, radioaktive Marker, enzymatische Marker, Latex-Partikel, photonische Kristalle und kolloidales Gold.

**18.** Verfahren nach Anspruch 11, in dem die Bestimmung des Signals jedes Platzes der Referenzskala und/oder jedes Analyseplatzes durch eine Methode realisiert wird, die ausgewählt wird aus der Gruppe, welche die Techniken der Detektion durch plasmonische Oberflächenresonanz, die photothermischen Detektionstechniken, die ellipsometrischen Techniken und die photometrischen Detektionstechniken umfasst.

**19.** Verfahren nach Anspruch 11, in dem die Analyse eine Detektion oder eine Dosierung ist.

FIG. 1

FIG. 2

FIG. 3

FIG. 4

FIG. 5

FIG. 6

FIG. 7

FIG. 8

FIG. 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1119769 A
- FR 2784189 A
- WO 03023061 A
- FR 2799281 A
- FR 2799282 A
- FR 9807870

- EP 1238116 A, A. Banerjee, A. Laayoun, M. Becker, K. Browne, M. Friedenberg, F. Hajjar.
- WO 0144506 A, A. Laayoun, D. Do, C. Miyada
- FR 0106039, J. Lhomme, E. Trevisiol, A. Laayoun, C. Bourget, M. Kotera, C. Tora

**Littérature non-brevet citée dans la description**

- **FRANÇOIS GELI.** Puces à ADN et autres systèmes d'analyse. *Biofutur n°206, Le technoscope de Biofutur,* Décembre 2000, 1-14
- **CAHILL DJ ; NORDHOFF E.** protein arrays and their role in proteomics. *Adv. Biochem. Eng. Biotechnol.,* 2003, vol. 83, 177-187
- **LOPEZ MF ; PLUSKAL MG.** Protein micro- and macroarrays : digitizing the proteome. *J. Chromatogr. B Analyt. Technol. Biomed. Life Sci.,* 2003, vol. 787 (1), 19-27
- Bar-coding biomolecules with fluorescent nanocrystals. *Nature Biotechnology,* 2001, vol. 19, 621-622

- **BERTRAND JORDAN.** voyage au pays des puces. *médecine/sciences,* 1998, vol. 14, 1097-1102
- **TROESCH A. et al.** Mycobacterium species identification and rifampin resistance testing with high-density DNA probe array. *J. Clin. Microbiol.,* 1999, vol. 37, 49-55
- **CHRISTEN R. ; MABILAT C.** Applications des puces à ADN en bactériologie. *Bull. Soc. Fr. Microbiol.,* 1998, vol. 13, 10-17
- NORMALIZATION STRATEGIES FOR CDNA MICROARRAYS. **SCHUCHHARDT J et al.** NUCLEIC ACIDS RESEARCH. OXFORD UNIVERSITY PRESS, 15 Mai 2000, vol. 28, E47I-E47V